(19) Europäisches Patentamt
European Patent Office
Office européen des brevets

(11) EP 1 109 810 B1

(12) # EUROPÄISCHE PATENTSCHRIFT

(45) Veröffentlichungstag und Bekanntmachung des Hinweises auf die Patenterteilung:
**16.06.2004 Patentblatt 2004/25**

(21) Anmeldenummer: **99968237.0**

(22) Anmeldetag: **21.08.1999**

(51) Int Cl.[7]: **C07D 471/04**, A61K 31/4745

(86) Internationale Anmeldenummer:
**PCT/EP1999/006139**

(87) Internationale Veröffentlichungsnummer:
**WO 2000/012501 (09.03.2000 Gazette 2000/10)**

(54) **BENZONAPHTHYRIDIN-N-OXIDE MIT PDE3 UND PDE4 INHIBIERENDER AKTIVITÄT**

BENZONAPHTHYRIDINE-N-OXIDES COMPRISING A PDE3 AND PDE4 INHIBITING ACTIVITY

N-OXYDES DE BENZONAPHTHYRIDINE POSSEDANT UNE ACTIVITE ANTI-PDE3 ET ANTI-PDE4

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **31.08.1998 EP 98116416**

(43) Veröffentlichungstag der Anmeldung:
**27.06.2001 Patentblatt 2001/26**

(73) Patentinhaber: **ALTANA Pharma AG**
**78467 Konstanz (DE)**

(72) Erfinder:
- **SCHMIDT, Beate**
  **D-78476 Allensbach (DE)**
- **AMSCHLER, Hermann**
  **(DE)**
- **ULRICH, Wolf-Rüdiger**
  **D-78464 Konstanz (DE)**
- **MARTIN, Thomas**
  **D-78464 Konstanz (DE)**
- **BÄR, Thomas**
  **D-78479 Reichenau (DE)**
- **HATZELMANN, Armin**
  **D-78467 Konstanz (DE)**
- **BOSS, Hildegard**
  **D-78464 Konstanz (DE)**
- **BEUME, Rolf**
  **D-78465 Konstanz (DE)**
- **BUNDSCHUH, Daniela**
  **D-78462 Konstanz (DE)**
- **KLEY, Hans-Peter**
  **D-78476 Allensbach (DE)**
- **FLOCKERZI, Dieter**
  **D-78476 Allensbach (DE)**

(56) Entgegenhaltungen:
**WO-A-98/21208**

EP 1 109 810 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Anwendungsgebiet der Erfindung

Die Erfindung betrifft neue Benzonaphthyridin-N-Oxide, die in der pharmazeutischen Industrie zur Herstellung von Medikamenten verwendet werden.

Bekannter technischer Hintergrund

In der DE-OS 21 23 328 und im USP 3,899,494 werden substituierte Benzonaphthyridine beschrieben, die sich durch ausgeprägte Blutplättchenaggregationshemmung auszeichnen. In den internationalen Anmeldungen WO91/17991 und WO98/21208 werden 6-Phenylbenzonaphthyridine zur Behandlung entzündlicher Atemwegserkrankungen offenbart.

Beschreibung der Erfindung

Es wurde nun gefunden, daß die nachfolgend näher beschriebenen Verbindungen der Formel I, die sich von den Verbindungen der WO91/17991 bzw. WO98/21208 insbesondere durch die Substitution am 6-Phenylring und das Vorhandensein eines N-Oxids in 2-Position unterscheiden, überraschende und besonders vorteilhafte Eigenschaften besitzen.

Gegenstand der Erfindung sind somit Verbindungen der Formel I,

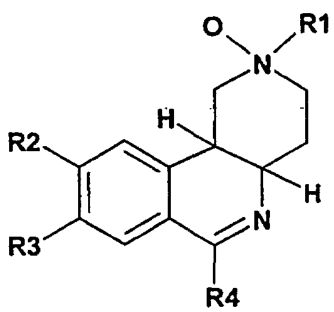

(I)

worin

R1 1-4C-Alkyl bedeutet,

R2 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,

R3 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,

oder worin

R2 und R3 gemeinsam eine 1-2C-Alkylendioxygruppe bedeuten,

R4 einen durch R5 substituierten Phenylrest darstellt, wobei

R5 einen durch einen Rest R6 substituierten Tetrazol-5-ylrest darstellt, wobei

R6 Wasserstoff, 1-10C-Alkyl, 3-7C-Cycloalkyl, 3-7C-Cycloalkylmethyl oder Ar-1-4C-alkyl bedeutet, wobei

Ar einen unsubstituierten oder durch R7 und/oder R8 substituierten Phenylrest darstellt, und

R7 und R8 unabhängig voneinander 1-4C-Alkyl oder 1-4C-Alkoxy bedeuten, sowie die Salze dieser Verbindungen.

1-4C-Alkyl steht für einen geradkettigen oder verzweigten Alkylrest mit 1 bis 4 Kohlenstoffatomen. Beispielsweise seien genannt der Butyl-, iso-Butyl-, sec.-Butyl-, tert.-Butyl-, Propyl-, Isopropyl- und bevorzugt der Ethyl- und Methylrest.

1-4C-Alkoxy steht für Reste, die neben dem Sauerstoffatom einen geradkettigen oder verzweigten Alkylrest mit 1 bis 4 Kohlenstoffatomen enthalten. Beispielsweise seien genannt der Butoxy-, iso-Butoxy-, sec.-Butoxy-, tert. -Butoxy-, Propoxy-, Isopropoxy- und bevorzugt der Ethoxy- und Methoxyrest.

3-7C-Cycloalkoxy steht für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder überwiegend durch Fluor substituiertes 1-4C-Alkoxy seien beispielsweise der 2,2,3,3,3-Pentafluorpropoxy-, der Perfluorethoxy-, der 1,2,2-Trifluorethoxy-, insbesondere der 1,1,2,2-Tetrafluorethoxy-, der Trifluormethoxy-, der 2,2,2-Trifluorethoxy- und bevorzugt der Difluormethoxyrest genannt.

1-2C-Alkylendioxy steht beispielsweise für den Methylendioxy- ($-O-CH_2-O-$) und den Ethylendioxyrest ($-O-CH_2-CH_2-O-$).

1-10C-Alkyl steht für geradkettige oder verzweigte Alkylreste mit 1 bis 10 Kohlenstoffatomen. Beispielsweise seien genannt der Decyl-, Nonyl-, Octyl-, Heptyl-, Isoheptyl- (5-Methylhexyl-), Hexyl-, Isohexyl- (4-Methylpentyl-), Neohexyl- (3,3-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkyl steht für den Cyclopropyl-, Cyclobutyl-, Cyclopentyl-, Cyclohexyl- und Cycloheptylrest. Bevorzugt seien die 5-7C-Cycloalkylreste Cyclopentyl, Cyclohexyl und Cycloheptyl genannt.

3-7C-Cycloalkylmethyl steht für einen Methylrest, der durch einen der vorstehend genannten 3-7C-Cycloalkylreste substituiert ist. Beispielsweise seien genannt der Cyclopentylmethyl- und der Cyclohexylmethylrest.

Ar-1-4C-alkyl steht für einen der vorstehend genannten 1-4C-Alkylreste, der durch einen der vorstehend definierten Arylreste substituiert ist. Beispielsweise seien genannt der p-Methoxybenzyl-, der Phenethyl- und der Benzylrest.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren und Basen. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen - zum Beispiel im Fall einer 1H- oder 2H-Tetrazol-5-yl-Substitution - auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel I, sowie alle Solvate und insbesondere alle Hydrate der Salze der Verbindungen der Formel 1.

Hervorzuhebende Verbindungen der Formel I sind solche, worin

R1 1-2C-Alkyl bedeutet,
R2 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R3 1-4C-Alkoxy, 3-7C-Cycloalkoxy, 3-7C-Cycloalkylmethoxy oder ganz oder überwiegend durch Fluor substituiertes 1-4C-Alkoxy bedeutet, oder worin

R2 und R3 gemeinsam eine 1-2C-Alkylendioxygruppe bedeuten,

R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, 3-7C-Cycloalkyl, 3-7C-Cycloalkylmethyl oder Ar-1-4C-alkyl bedeutet, wobei
Ar einen unsubstituierten oder durch R7 und/oder R8 substituierten Phenylrest darstellt, und

R7 und R8 unabhängig voneinander 1-4C-Alkyl oder 1-4C-Alkoxy bedeuten,

sowie die Salze dieser Verbindungen.

Besonders hervorzuhebende Verbindungen der Formel I sind solche, in denen

R1 Methyl bedeutet,
R2 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R3 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, 5-7C-Cycloalkyl, 3-7C-Cycloalkylmethyl oder Ar-1-2C-alkyl bedeutet, wobei
Ar einen unsubstituierten oder durch R7 substituierten Phenylrest darstellt, und
R7 1-2C-Alkyl oder 1-2C-Afkoxy bedeutet,

sowie die Salze dieser Verbindungen.

Bevorzugte Verbindungen der Formel I sind solche, in denen

R1 Methyl bedeutet,
R2 1-4C-Alkoxy bedeutet,
R3 1-4C-Alkoxy bedeutet,
R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, Cyclohexylmethyl oder 4-Methoxybenzyl bedeutet,

sowie die Salze dieser Verbindungen.

Besonders bevorzugte Verbindungen der Formel I sind solche, in denen

R1 Methyl bedeutet,
R2 Ethoxy bedeutet,
R3 Methoxy oder Ethoxy bedeutet,
R4 einen durch R5 substituierten Phenylrest darstellt, wobei
R5 einen durch einen Rest R6 substituierten Tetrazol-5-ylrest darstellt, wobei
R6 1-4C-Alkyl bedeutet,

sowie die Salze dieser Verbindungen.

Bei den Verbindungen der Formel I handelt es sich um chirale Verbindungen mit Chiralitätszentren in den Positionen 2, 4a und 10b. Die Bezifferung (Numerierung) der Verbindungen der Formel I ist in Formel 1a dargestellt.

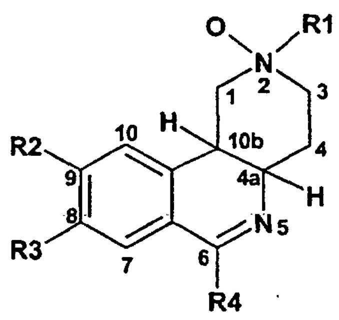

(Ia)

Gegenstand der Erfindung sind alle acht denkbaren Enantiomeren in jedem beliebigen Mischungsverhältnis zueinander. Bevorzugt sind die Verbindungen der Formel I, in denen die Wasserstoffatome in den Positionen 4a und 10b cis-ständig zueinander sind.

Besonders bevorzugt sind in diesem Zusammenhang solche Verbindungen der Formel I, die in den Positionen 4a und 10b dieselbe absolute Konfiguration haben wie die als Ausgangsprodukt einsetzbare Verbindung (-)-cis-4-Amino-3-(3-ethoxy-4-methoxyphenyl)-1-methylpiperidin Dihydrochlorid mit dem optischen Drehwert $[\alpha]_D^{20}$ = -65,5° (c=1, Methanol).

Der Tetrazol-5-ylrest R5 der Verbindungen der Formel I kann am Phenylrest R4 sowohl in ortho-, meta-, als

auch in para-Position zum Benzonaphthyridinring angebunden sein.

Bevorzugt sind diejenigen Verbindungen der Formel I, bei denen der Tetrazol-5-ylrest R5 in metaoder para-Position zum Benzonaphthyridinring am Phenylrest R4 angebunden ist. Insbesondere bevorzugt sind in diesem Zusammenhang die Verbindungen der Formel I, bei denen der Tetrazol-5-ylrest R5 in para-Position angebunden ist.

Verbindungen der Formel I, bei denen R1, R2, R3, R4 und R5 die oben angegebenen Bedeutungen haben und R6 für Wasserstoff steht, treten in mehreren tautomeren Formen auf, die miteinander im Gleichgewicht stehen (z. B. 1H- und 2H-Form des Tetrazol-5-ylrestes). Die Erfindung umfaßt alle tautomeren Formen in jedem Mischungsverhältnis.

Durch Anbindung der Substituenten R6 (R6≠H) an die Tetrazol-5-yl-gruppe wird die Umwandlung der beiden tautomeren 1H- und 2H-Formen des Tetrazol-5-ylrestes ineinander blockiert. Gegenstand der Erfindung sind daher auch die durch einen Rest R6 (R6≠H) substituierten 1H- und 2H-Tetrazol-5-ylverbindungen der Formel I, sowohl in reiner Form, als auch in jedem Mischungsverhältnis. Bevorzugt sind jedoch die Verbindungen der Formel I, bei denen der Tetrazol-5-ylrest in 2-Position durch einen der Reste R6 (R6≠H) substituiert ist.

Weiterer Gegenstand der Erfindung ist ein Verfahren zur Herstellung der Verbindungen der Formel I, worin R1, R2, R3 und R4 die oben angegebenen Bedeutungen besitzen, und ihrer Salze. Das Verfahren ist dadurch gekennzeichnet, daß man Verbindungen der Formel II,

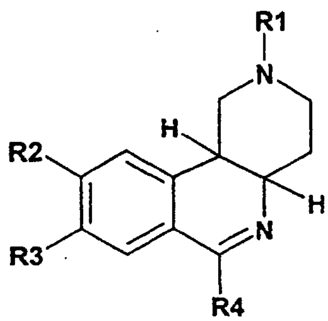

(II)

in denen R1, R2, R3 und R4 die oben angegebenen Bedeutungen besitzen, einer N-Oxidation unterwirft und gewünschtenfalls anschließend die erhaltenen Verbindungen der Formel I in ihre Salze überführt, oder daß man gewünschtenfalls anschließend erhaltene Salze der Verbindungen der Formel I in die freien Verbindungen überführt.

Die N-Oxidation erfolgt auf eine dem Fachmann vertraute Weise, z.B. mit Hilfe von Wasserstoffperoxid in Methanol oder mit Hilfe von m-Chforperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Verbindungen der Formel II, worin R1, R2 und R3 die oben angegebenen Bedeutungen haben, R4 einen durch einen 1H- oder 2H-Tetrazol-5-ylrest R5 substituierten Phenylrest darstellt, können beispielsweise aus den entsprechenden Verbindungen der Formel II, in denen R4 einen durch eine Cyanogruppe substituierten Phenylrest darstellt, durch Umsetzung mit einem Alkalimetallazid und einem Ammoniumhalogenid (z.B. Ammoniumchlorid) hergestellt werden. Entsprechende Reaktionen sind z.B. in J. Med. Chem. **1993,** 36, 3246 beschrieben.

Die auf diese Weise erhaltenen Verbindungen der Formel II können gewünschtenfalls durch eine Alkylierungsreaktion in weitere Verbindungen der Formel II übergeführt werden, wobei der Wasserstoff am Tetrazol-5-ylrest durch einen der für R6 oben genannten Reste - ausgenommen Wasserstoff - ersetzt wird.

Die Alkylierungsreaktionen erfolgen zweckmäßigerweise analog zu den dem Fachmann bekannten Methoden, z.B. durch Reaktion der 1H- oder 2H-Tetrazolverbindungen der Formel II mit Verbindungen der Formel R6-X in Gegenwart einer Base, wobei R6 die oben genannten Bedeutungen - ausgenommen Wasserstoff - hat und X eine geeignete Abgangsgruppe wie beispielsweise ein Chlor-, Bromoder Jodatom oder einen Alkylsulfatrest darstellt. Die bei der Alkylierung gewöhnlich entstehenden 1-und 2-substituierten Tetrazolregioisomerengemische werden durch dem Fachmann bekannte Methoden wie Kristallisation oder Chromatographie an geeigneten Trägermaterialien getrennt. Eine analoge Alkylierung von Tetrazolen und Trennung der Regioisomeren ist beispielsweise in J. Med. Chem. 1996, 39, 2354 beschrieben.

Verbindungen der Formel II, worin R1, R2 und R3 die oben angegebenen Bedeutungen haben und R4 einen durch einen Tetrazol-5-ylrest R5 substituierten Phenylrest darstellt, wobei der Tetrazol-5-ylrest R5 seinerseits durch R6 (R6 ≠ Wasserstoff) substituiert ist, können alternativ auch durch eine Cyclokondensationsreaktion der entsprechenden Verbindungen der Formel III

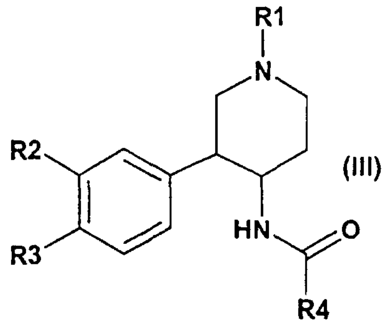

(III)

erhalten werden.

Die Cyclokondensation erfolgt auf eine dem Fachmann an sich bekannte Weise gemäß Bischler-Napieralski (z.B. so, wie in J. Chem. Soc., **1956**, 4280-4282 beschrieben) in Gegenwart eines geeigneten Kondensationsmittels, wie beispielsweise Polyphosphorsäure, Phosphorpentachlorid, Phosphortrichlorid, Phosphorpentoxid , Thionylchlorid oder bevorzugt Phosphoroxytrichlorid, in einem geeigneten inerten Lösungsmittel, z.B. in einem chlorierten Kohlenwasserstoff wie Chloroform, oder in einem cyclischen Kohlenwasserstoff wie Toluol oder Xylol, oder einem sonstigen inerten Lösungsmittel wie Acetonitril, oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Enantiomerenreine Verbindungen der Formel II können in bekannter Weise (beispielsweise durch Herstellung und Trennung entsprechender diastereoisomerer Verbindungen) separiert oder durch stereoselektive Synthesemethoden hergestellt werden. Solche Trennverfahren und Synthesemethoden sind beispielsweise in der EP 247 971 und in der DE 42 17 401 beschrieben.

Verbindungen der Formel III, worin R1, R2, R3 und R4 die oben angegebenen Bedeutungen haben sind aus den entsprechenden Verbindungen der Formel IV,

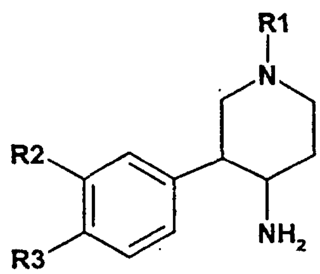

(IV)

worin R1, R2 und R3 die oben angegebenen Bedeutungen haben, durch Umsetzung mit Verbindungen der Formel R4-CO-Y, worin R4 die oben angegebenen Bedeutungen hat und Y eine geeignete Abgangsgruppe, vorzugsweise ein Chloratom darstellt, zugänglich. Beispielsweise wird die Benzoylierung wie in den nachfolgenden Beispielen nach dem Einhorn-Verfahren, der Schotten-Baumann-Variante oder wie in J. Chem. Soc. (C), **1971**, 1805-1808 beschrieben, durchgeführt.

Verbindungen der Formel R4-CO-Y sind entweder bekannt oder können durch Umsetzung auf eine dem Fachmann geläufige Weise aus den entsprechenden Carbonsäuren R4-COOH, worin R4 die oben angegebenen Bedeutungen hat, hergestellt werden.

Die Verbindungen R4-COOH, worin R4 die oben angegebenen Bedeutungen hat, sind entweder bekannt oder können auf eine dem Fachmann bekannte Weise aus 2-, 3- oder 4-Cyanbenzoesäurealkylestern erhalten werden, z.B. durch Umsetzung mit Alkaliaziden und einem Ammoniumhalogenid (z.B. Ammoniumchlorid) zu im Tetrazolteil unsubstituierten 2-, 3- oder 4-(1H- oder 2H-Tetrazol-5-yl)benzoesäurealkylestern. Eine solche Umsetzung ist beispielsweise in J. Med. Chem. **1993**, 36, 3246 beschrieben. Gewünschtenfalls lassen sich diese Zwischenverbindungen - wie oben für die 1 H- oder 2H-Tetrazolverbindungen der Formel II oder in der vorstehend genannten Literatur beschrieben - durch Alkylierung mit Verbindungen der Formel R6-X in Gegenwart einer Base in R4-Carbonsäurealkylester überführen, in denen R4 einen durch R5 substituierten Phenylrest darstellt, R5 einen durch einen Rest R6 substituierten

1H- oder 2H-Tetrazol-5-ylrest darstellt und R6 nicht Wasserstoff ist, sondern eine der anderen für R6 oben genannten Bedeutungen hat. Durch dem Fachmann geläufige alkalische oder saure Hydrolysebedingungen werden die R4-Carbonsäurealkylester in die freien Carbonsäuren R4-COOH übergeführt.

Die Herstellung von cis/trans-Racemat-Gemischen und von reinen cis-Racematen von Verbindungen der Formel IV ist beispielsweise im USP 3,899,494, in DE-OS 21 23 328 und in DE-OS 16 95 782 beschrieben. Reine cis-Enantiomere der Verbindungen der Formel IV können beispielsweise nach den Verfahren erhalten werden, wie sie in EP 0 247 971 und in DE 42 17 401 offenbart sind.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel (z. B. einem Keton, wie Aceton, Methylethylketon oder Methylisobutylketon, einem Ether, wie Diethylether, Tetrahydrofuran oder Dioxan, einem chlorierten Kohlenwasserstoff, wie Methylenchlorid oder Chloroform, oder einem niedermolekularen aliphatischen Alkohol wie Ethanol oder Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die nachfolgenden Beispiele dienen der näheren Erläuterung der Erfindung, ohne sie einzuschränken. Ebenso können weitere Verbindungen der Formel I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, h für Stunde(n), RT für Raumtemperatur, SF für Summenformel, MG für Molgewicht, DC für Dünnschichtchromatographie, Ber. für Berechnet, Gef. für Gefunden. Die in den Beispielen genannten Verbindungen und ihre Salze sind bevorzugter Gegenstand der Erfindung.

## Beispiele

## Endprodukte

### 1. cis-9-Ethoxy-8-methoxy-2-methyl-6-[4-(2H-2-ethyltetrazol-5-yl)phenyl]-1,2,3,4,4a,10b-hexahydrobenzo[c][1,6]naphthyridin-N-2-oxid

Eine Lösung von 2,23 g (-)-cis-9-Ethoxy-8-methoxy-2-methyl-6-(4-(2H-2-ethyftetrazol-5-yl)phenyl]-1,2,3,4,4a,10b-hexahydrobenzo[c][1,6]naphthyridin (Ausgangsverbindung A) in 12 ml Methanol wird mit 6 ml 30 % Wasserstoffperoxid ca. 2 Tage bei RT gerührt. Nach vollständiger Oxidation (DC-Kontrolle) versetzt man das Reaktionsgemisch mit 7 g festem Natriumsulfit und rührt noch ca. 1 h bei RT nach. Nach dem Absaugen des Reaktionsgemisches extrahiert man das Filtrat mit Dichlormethan, wäscht die organische Phase mit Wasser und trocknet sie über Natriumsulfat. Nach dem Absaugen und Einengen der Produktlösung kristallisiert man den erhaltenen festen Rückstand in einem Ethylacetat/Diethylether-Gemisch (2:1) um. Man erhält 1,9 g der Titelverbindung als farblose feine Kristalle vom Schmp. 168 - 170° C.
SF: $C_{25}$ $H_{30}N_6$ $O_3$ x 1,19 $H_2O$; MG: 484,07

| | | | | |
|---|---|---|---|---|
| Elementaranalyse | Ber. | C 62,14 | H 6.74 | N 17,39 |
| | Gef. | C 62,18 | H 7.04 | N 17,44 |

## Ausgangsverbindungen:

### A. (-)-cis-9-Ethoxy-8-methoxy-2-methyl-6-[4-(2H-2-ethyltetrazol-5-yl)phenyl]-1,2,3,4,4a,10bhexahydrobenzo[c][1,6]naphthyridin

6,7 g (-)-cis-3-(3-Ethoxy-4-methoxyphenyl)-4-[4-(2H-2-ethyltetrazol-5-yl)-benzamido]-1-methylpiperidin werden in 20 ml Phosphoroxytrichlorid und 80 ml Acetonitril 16 h unter Rückfluß zum Sieden erhitzt. Nach Abdestillieren des überschüssigen Phosphoroxytrichlorids verteilt man den Rückstand zwischen Dichlormethan und gesättigter Natriumhydrogencarbonatlösung. Die organische Phase wird mit Wasser gewaschen, über Natriumsulfat getrocknet und eingeengt. Den festen Rückstand reinigt man durch Kieselgelchromatographie, separiert die Hauptproduktfraktion und

engt diese ein. Der Rückstand wird aus Petrolether/Diethylether (2:1) umkristallisiert. Man erhält 4,6 g der Titelverbindung (hellgelbe Kristalle) vom Schmp. 151-152°C.
SF: $C_{25}H_{30}N_6O_2$, MG: 503,44
Optischer Drehwert: $[\alpha]_D^{20}$ = -106.4° (c=1, Methanol)

**B. (-)-cis-3-(3-Ethoxy-4-methoxyphenyl)-4-[4-(2H-2-ethyltetrazol-5-yl)-benzamido]-1-methylpiperidin**

Zur im Eis-/Wasserbad gekühlten Lösung von 1,82 g (-)-cis-4-Amino-3-(3-ethoxy-4-methoxyphenyl)-1-methylpiperidin (freie Base, hergestellt durch Behandlung des Dihydrochlorids mit Natronlauge und Extraktion der freien Base mit Dichlormethan) in 60 ml Dichlormethan und 1 ml Triethylamin wird eine Lösung von 4-(2H-2-Ethyltetrazol-5-yl)benzoylchlorid (hergestellt durch Erhitzen unter Rückfluß von 1,5 g 4-(2H-2-Ethyltetrazol-5-yl)-benzoesäure mit 2 ml Thionylchlorid in 60 ml absolutem Toluol für ca. 2 h und vollständigem Einengen) innerhalb von 10 Min zugetropft. Das Reaktionsgemisch wird unter Rühren auf RT erwärmt und noch ca. 2 h nachgerührt. Nach Extraktion mit einem Gemisch aus gesättigter Natriumhydrogencarbonat-Lösung und Dichlormethan engt man die organische Phase vollständig ein und kristallisiert den Rückstand in Methanol/Diethylether (1+1) um. Man erhält 3,15 g farblose Kristalle der Titelverbindung vom Schmp. 165-167,5°C.
SF: $C_{25}H_{32}N_6O_3$, MG: 464,57
Optischer Drehwert: $[\alpha]_D^{20}$ = -89,7° (c=1, Methanol)

| | | | | |
|---|---|---|---|---|
| Elementaranalyse | Ber. | C 64,64 | H 6,94 | N 18,09 |
| | Gef. | C 64,74 | H 7,08 | N 18,21 |

**C. (-)-cis-4-Amino-3-(3-ethoxy-4-methoxyphenyl)-1-methylpiperidin Dihydrochlorid**

Die Titelverbindung wird analog dem in DE 42 17 401 für (-)-cis-4-Amino-3-(3,4-dimethoxyphenyl)-1-methylpiperidin Dihydrochlorid beschriebenen Verfahren erhalten, wenn in den dort beschriebenen Beispielen die entsprechenden 3-Ethoxy-4-methoxyverbindungen eingesetzt werden.
SF: $C_{15}H_{24}N_2O_2$ x 2HCl x 0,96 $H_2O$, MG: 354,52; Schmp. 252-254°C
Optischer Drehwert: $[\alpha]_D^{20}$ = -65.5° (c=1, Methanol)

**Gewerbliche Anwendbarkeit**

Die erfindungsgemäßen Verbindungen besitzen wertvolle pharmakologische Eigenschaften, die sie gewerblich verwertbar machen. Als selektive Inhibitoren des Typs 3 und 4 der Zyklisch-Nukleotid-Phosphodiesterase (PDE3, PDE4) eignen sie sich einerseits als Bronchialtherapeutika (zur Behandlung von Atemwegsobstruktionen aufgrund ihrer dilatierenden und zilienstimulierenden aber auch aufgrund ihrer atemfrequenz- bzw. atemantriebssteigernden Wirkung) andererseits jedoch vor allem zur Behandlung von Erkrankungen entzündlicher Natur, z.B. der Atemwege (Asthma-Prophylaxe). der Haut, des Darms, der Augen und der Gelenke, die vermittelt werden durch Mediatoren wie Interferone, Mitglieder der Tumor Nekrose-Faktorenfamilie, Interleukine, Chemokine, Kolonie-stimulierende Faktoren, Wachstumsfaktoren, Lipidmediatoren (z.B. u.a. PAF, plättchenaktivierender Faktor), bakterielle Faktoren (z.B. LPS), Immunglobuline, Sauerstoffradikale und Verwandte (z.B. Stickstoffmonoxid NO), biogene Amine (z.B. Histamin, Serotonin), Kinine (z.B. Bradykinin), neurogene Mediatoren (wie Substanz P, Neurokinin), Proteine wie z.B. Granulainhaltsstoffe von Leukozyten (u.a. kationische Proteine von Eosinophilen) und Adhärenzproteine (z.B. Integrine). Die erfindungsgemäßen Verbindungen besitzen glattmuskelzell-relaxierende Wirkung, z.B. im Bereich des Bronchialsystems, des Blutkreislaufs, und der ableitenden Hamwege. Desweiteren besitzen sie Zilienfrequenz-steigernde Wirkung, z.B. im Bronchialsystem.

Hierbei zeichnen sich die erfindungsgemäßen Verbindungen durch eine geringe Toxizität, eine gute Humanakzeptanz, eine gute enterale Resorption und eine hohe Bioverfügbarkeit, eine große therapeutische Breite, das Fehlen wesentlicher Nebenwirkungen und eine gute Wasserlöslichkeit aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophyläxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiate, Emphysema, COPD); Erkrankungen mit einer Einschränkung der Zilientätigkeit oder verstärkten Anforderungen an die ziliäre Clearance (Bronchitis, Mucoviscidose), Dermatosen (vor allem profiferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis, der Arthritis (Rheumatoide Arthritis, Rheumatoide Spondylitis, Osteoarthritis und andere arthritische Zustände), systemischer Lupus erythematosus, Erkrankungen des Immunsystems (AIDS), einschließlich AIDS-bedingter Enzephalopathien, Autoimmun-Erkrankungen wie Diabetes mellitus (Typ I, Autoimmundiabetes), Multiple Sklerose und der Formenkreis Virus-, Bakterien- oder Parasiten-induzierter Demyelinisierungs-Krankheiten, zerebrale Malaria oder Lymes Erkrankung, Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; ferner Erkrankungen des Zentralnerversystems wie Gedächtnisstörungen und Alzheimer's Erkrankung, Candidiasis, Leishmaniosen und Lepra.

Aufgrund ihrer gefäßrelaxierenden Wirksamkeit können die erfindungsgemäßen Verbindungen auch zur Behandlung von Bluthochdruckerkrankungen verschiedener Genese wie z.B. pulmonarer Hochdruck und den damit zusammenhängenden Begleiterscheinungen, zur Behandlung erektiler Dysfunktion oder Koliken der Nieren und der Harnleiter im Zusammenhang mit Nierensteinen, verwendet werden.

Aufgrund ihrer cAMP-steigernden Wirkung können sie aber auch für Erkrankungen des Herzens, die durch PDE-Hemmstoffe behandelt werden können, wie beispielsweise Herzinsuffizienz, sowie als antithrombotische, die Plättchen-Aggregation hemmende Substanzen verwendet werden.

Weiterer Gegenstand der Erfindung sind die erfindungsgemäßen Verbindungen zur Anwendung bei der Behandlung und/oder Prophylaxe von Krankheiten, insbesondere den genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Ein weiterer Gegenstand der Erfindung ist ein Handelsprodukt, bestehend aus einem üblichen Sekundärpackmittel, einem das Arzneimittel enthaltenden Primärpackmittel (beispielsweise eine Ampulle oder ein Blister) und gewünschtenfalls einem Beipackzettel, wobei das Arzneimittel antagonistische Wirkung gegen zyklisch-nukleotid Phosphodiesterasen des Typs 3 und 4 zeigt und zur Abschwächung der Symptome von Krankheiten führt, die in Zusammenhang mit zyklisch-nukleotid Phosphodiesterasen des Typs 3 und 4 stehen, und wobei auf dem Sekundärpackmittel und/oder auf dem Beipackzettel des Handelsprodukts auf die Eignung des Arzneimittels zur Prophylaxe oder Behandlung von Krankheiten, die im Zusammenhang mit zyklisch-nukleotid Phosphodiesterasen des Typs 3 und 4 stehen, hingewiesen wird, und wobei das Arzneimittel ein oder mehrere erfindungsgemäße Verbindungen der Formel I enthält. Das Sekundärpackmittel, das das Arzneimittel enthaltende Primärpackmittel und der Beipackzettel entsprechen ansonsten dem, was der Fachmann als Standard für Arzneimittel dieser Art ansehen würde.

In vorteilhafter Weise eignen sich die erfindungsgemäßen Substanzen auch zur Kombination mit anderen Substanzen, die eine Stimulation von cAMP bewirken, wie Prostaglandine (PGE2, PG12 bzw. Prostazyklin) und ihre Abkömmlinge, direkte Adenylatzyklase-Stimulatoren wie Forskolin und verwandte Substanzen, bzw. mittelbar die Adenylatzyklase stimulierende Substanzen wie Katecholamine und adrenerge Rezeptoragonisten, insbesondere Beta-Mimetika. Sie entfalten dabei in Kombination auf Grund ihrer den cAMP-Abbau hemmenden Wirkung eine synergistische, überadditive Wirksamkeit. Diese kommt z.B. bei ihrer Anwendung in Kombination mit PGE2 zur Behandlung der pulmonalen Hypertonie zum Tragen.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnem, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiförmulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,1 und 3 mg pro Tag. Die übliche Dosis bei systemischer Therapie (p.o. oder i.v.) liegt zwischen 0,01 und 10 mg pro Kilogramm und Tag.

## Biologische Untersuchungen

Bei der Untersuchung der PDE4-Hemmung auf zellulärer Ebene kommt der Aktivierung von Entzündungszellen besondere Bedeutung zu. Als Beispiel sei die FMLP (N-formyl-methionylleucylphenylalanin)-induzierte Superoxid-Produktion von neutrophilen Granulozyten genannt, die als Luminol-verstärkte Chemilumineszenz gemessen werden kann. [Mc Phail LC, Strum SL, Leone PA und Sozzani S, The neutrophil respiratory burst mechanism. in "Immunology Series" **1992**, 57, 47-76; ed. Coffey RG (Marcel Decker, Inc., New York-Basel-Hong Kong)].

Substanzen, welche die Chemilumineszenz sowie die Zytokinsekretion und die Sekretion entzündungssteigemder Mediatoren an Entzündungszellen, insbesonders neutrophilen und eosinophilen Granulozyten, T-Lymphozyten, Monozyten und Makrophagen hemmen, sind solche, welche die PDE4 oder PDE3 und PDE4 hemmen. Das letztgenannte Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE4-Hemmung durch die erfindurigsgemäßen Substanzen ist damit ein zentraler Indikator für die Unterdrückung von entzündlichen Prozessen. (Giembycz MA, Could isoenzyme-selective phosphodiesterase inhibitors render bronchodilatory therapy redundant in the treatment of bronchial asthma?; Biochem Pharmacol **1992**, 43, 2041-2051; **Torphy TJ** et al., Phosphodiesterase inhibitors: new opportunities for treatment of asthma. Thorax **1991**, 46, 512-523; **Schudt C** et al., Zardaverine: a cyclic AMP PDE3/4 inhibitor. In "New Drugs for Asthma Therapy", 379-402, Birkhäuser Verlag Basel 1991; Schudt C et al., Influence of selective phosphodiesterase inhibitors on human neutrophil functions and levels of cAMP and Ca; Naunyn-Schmiedebergs Arch Pharmacol **1991**, 344, 682-690; **Tenor H** and Schudt C, Analysis of PDE isoenzyme profiles in cells and tissues by pharmacological methods. In "Phosphodiesterase Inhibitors", 21-40, "The Handbook of Immunopharmacology", Academic Press 1996. Hatzelmann A et al., Enzymatic and functional aspects of dual-selective PDE3/4-inhibitors. In "Phosphodiesterase Inhibitors", 147-160, "The Handbook of Immunopharmacology", Academic Press, 1996.

A. Methodik

1. Hemmung der PDE Isoenzyme

Die PDE-Aktivität wurde gemäß Thompson et al. (1) mit einigen Modifikationen (2) bestimmt. Die Prüfproben enthielten 40 mM Tris-HCl (pH 7,4), 5 mM $MgCl_2$, 0,5 μM cAMP oder cGMP, [$^3$H]cAMP oder [$^3$H]cGMP (ca. 50.000 cpm/Probe), die unten näher beschriebenen PDE Isoenzym-spezifischen Zusätze, die angegebenen Konzentrationen an Hemmstoff und ein Aliquot der Enzymlösung bei einem Gesamtprobenvolumen von 200 μl. Stammlösungen der zu untersuchenden Verbindungen in DMSO wurden in solchen Konzentrationen hergestellt, daß der DMSO-Gehalt - zur Vermeidung einer Beeinflussung der PDE-Aktivität - in den Prüfproben 1 Vol-% nicht überschritt. Nach 5-minütiger Vorinkubation bei 37°C wurde die Reaktion durch Zugabe des Substrates (cAMP oder cGMP) in Gang gesetzt. Die Proben wurden für weitere 15 Min. bei 37°C inkubiert. Durch Zugabe von 50 μl 0,2 N HCl wurde die Reaktion abgebrochen. Nach 10-minütiger Kühlung auf Eis und Zugabe von 25 μg 5'-Nukleotidase (Schlangengift von Crotalus atrox) wurde erneut für 10 Min. bei 37°C inkubiert und die Proben dann auf QAE Sephadex A-25-Säulen aufgetragen. Die Säulen wurden mit 2 ml 30 mM Ammoniumformiat (pH 6,0) eluiert. Die Radioaktivität des Eluats wurde gemessen und um die entsprechenden Leerwerte korrigiert. Der Anteil an hydrolysiertem Nucleotid überschritt in keinem Fall 20 % der ursprünglichen Substratkonzentration.

PDE1 ($Ca^{2+}$/Calmodulin-abhängig) aus Rinderhirn: Die Hemmung dieses Isoenzyms wurde in Gegenwart von $Ca^{2+}$ (1 mM) und Calmodulin (100 nM) unter Verwendung von cGMP als Substrat untersucht (3).

PDE2 (cGMP-stimuliert) aus Rattenherzen wurde chromatografisch gereinigt [Schudt et al. (4)] und in Gegenwart von cGMP (5 μM) unter Verwendung von cAMP als Substrat untersucht.

PDE3 (cGMP-inhibiert) und PDE5 (cGMP-spezifisch) wurden in Homogenaten von menschlichen Blutplättchen [Schudt et al. (4)] unter Verwendung von cAMP bzw. cGMP als Substrat untersucht.

PDE4 (cAMP-spezifisch) wurde im Zytosol von humanen polymorphonuclearen Leukozyten (PMNL) [isoliert aus Leukozytenkonzentraten, siehe Schudt et al. (5)] unter Verwendung von cAMP als Substrat untersucht. Der PDE3-Hemmstoff Motapizon (1 µM) wurde verwendet um die von verunreinigenden Blutplättchen ausgehende PDE3 Aktivität zu unterdrücken.

2. Statistik

Die $IC_{50}$-Werte wurden aus den Konzentrations-Hemmkurven durch nichtlineare Regression unter Verwendung des Programms GraphPad InPlot™ (GraphPad Software Inc., Philadelphia, USA) ermittelt.

3. Literatur

(1) Thompson W.J., Terasaki W. L., Epstein P. M. and Strada S. J., Assay of cyclic nucleotide phosphodiesterase and resolution of multiple molecular forms of the enzyme; Adv. Cycl. Nucl. Res. **1979,** 10, 69-92

(2) Bauer A.C. und Schwabe U., An improved assay of cyclic 3',5'-nucleotide phosphodiesterase with QAE Sephadex A-25; Naunyn-Schmiedeberg's Arch. Pharmacol. **1980**, 311, 193-198

(3) Gietzen K., Sadorf I. und Bader H., A model for the regulation of the calmodulin-dependent enzymes erythrocyte $Ca^{2+}$-transport ATPase and brain phosphodiesterase by activators and inhibitors; Biochem. J. **1982**, 207, 541-548

(4) Schudt C., Winder S., Müller B. und Ukena D., Zardaverine as a selective inhibitor of phosphodiesterase isoenzymes; Biochem. Pharmacol. **1991**, 42, 153-162

(5) Schudt C., Winder S., Forderkunz S., Hatzelmann A. und Ullrich V., Influence of selective phosphodiesterase inhibitors on human neutrophil functions and levels of cAMP and Ca; Naunyn-Schmiedeberg's Arch. Pharmacol. **1991**, 344, 682-690

B. Ergebnisse

In der nachfolgenden Tabelle 1 sind die gemäß Punkt A1 ermittelten Hemmkonzentrationen [Hemmkonzentrationen als -log $IC_{50}$ (mol/l)] für die Verbindung 1 für verschiedene PDE Isoenzyme angegeben. Die Nummer der Verbindung entspricht der Nummer des Beispiels im Abschnitt Endprodukte.

Tabelle 1

| Verbindung | PDE4 | PDE3 |
|---|---|---|
| | [-log $IC_{50}$, mol/l] | |
| 1 | 7,53 | 6,11 |

## Patentansprüche

**1.** Verbindungen der Formel I,

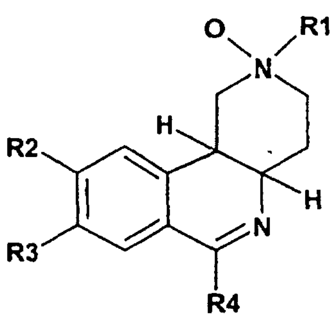

(I)

worin

R1 1-4C-Alkyl bedeutet,
R2 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R3 Hydroxy, 1-4C-Alkoxy, 3-7C-Cycloalkoxy, 3-7C-Cycloalkylmethoxy oder ganz oder überwiegend durch Fluor substituiertes 1-4C-Alkoxy bedeutet,

oder worin
R2 und R3 gemeinsam eine 1-2C-Alkylendioxygruppe bedeuten,

R4 einen durch R5 substituierten Phenylrest darstellt, wobei
R5 einen durch einen Rest R6 substituierten Tetrazol-5-ylrest darstellt, wobei
R6 Wasserstoff, 1-10C-Alkyl, 3-7C-Cycloalkyl, 3-7C-Cycloalkylmethyl oder Ar-1-4C-alkyl bedeutet, wobei
Ar einen unsubstituierten oder durch R7 und/oder R8 substituierten Phenylrest darstellt, und

R7 und R8 unabhängig voneinander 1-4C-Alkyl oder 1-4C-Alkoxy bedeuten,
sowie die Salze dieser Verbindungen.

**2.** Verbindungen der Formel I nach Anspruch 1, in denen

R1 1-2C-Alkyl bedeutet,
R2 1-4C-Alkoxy, 3-7C-Cycloalkoxy, 3-7C-Cycloalkylmethoxy oder ganz oder überwiegend durch Fluor substituiertes 1-4C-Alkoxy bedeutet,
R3 1-4C-Alkoxy, 3-7C-Cycloalkoxy, 3-7C-Cycloalkylmethoxy oder ganz oder überwiegend durch Fluor substituiertes 1-4C-Alkoxy bedeutet, oder worin

R2 und R3 gemeinsam eine 1-2C-Alkylendioxygruppe bedeuten,

R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, 3-7C-Cycloalkyl, 3-7C-Cycloalkylmethyl oder Ar-1-4C-alkyl bedeutet, wobei
Ar einen unsubstituierten oder durch R7 und/oder R8 substituierten Phenylrest darstellt, und

R7 und R8 unabhängig voneinander 1-4C-Alkyl oder 1-4C-Alkoxy bedeuten,
sowie die Salze dieser Verbindungen.

**3.** Verbindungen der Formel I nach Anspruch 1, in denen

R1 Methyl bedeutet,
R2 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R3 1-4C-Alkoxy, 3-7C-Cycloalkoxy, 3-7C-Cycloalkylmethoxy oder ganz oder überwiegend durch Fluor substituiertes 1-2C-Alkoxy bedeutet,
R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, 5-7C-Cycloalkyl, 3-7C-Cycloalkylmethyl oder Ar-1-2C-alkyl bedeutet, wobei
Ar einen unsubstituierten oder durch R7 substituierten Phenylrest darstellt, und
R7 1-2C-Alkyl oder 1-2C-Alkoxy bedeutet,

sowie die Salze dieser Verbindungen.

**4.** Verbindungen der Formel I nach Anspruch 1, in denen

R1 Methyl bedeutet,
R2 1-4C-Alkoxy bedeutet,
R3 1-4C-Alkoxy bedeutet,
R4 einen durch R5 substituierten Phenylrest darstellt, wobei
R5 einen durch einen Rest R6 substituierten Tetrazol-5-ylrest darstellt, wobei
R6 Wasserstoff, 1-7C-Alkyl, Cyclohexylmethyl oder 4-Methoxybenzyl bedeutet,

sowie die Salze dieser Verbindungen.

**5.** Verbindungen der Formel I nach Anspruch 1, in denen

R1 Methyl bedeutet,
R2 Ethoxy bedeutet,
R3 Methoxy oder Ethoxy bedeutet,
R4 einen durch R5 substituierten Phenylrest darstellt, wobei
R5 einen durch einen Rest R6 substituierten Tetrazol-5-ylrest darstellt, wobei
R6 1-4C-Alkyl bedeutet,

sowie die Salze dieser Verbindungen.

**6.** Verbindungen der Formel I nach Anspruch 1, in denen die Wasserstoffatome in den Positionen 4a und 10b cis-ständig zueinander sind, sowie die Salze dieser Verbindungen.

**7.** Verbindungen der Formel I nach Anspruch 1, die in den Positionen 4a und 10b dieselbe absolute Konfiguration haben, wie die als Ausgangsprodukt einsetzbare Verbindung (-)-cis-4-Amino-3-(3-ethoxy-4-methoxyphenyl)-1-methylpiperidin Dihydrochlorid mit dem optischen Drehwert $[\alpha]_D^{20}$ = -65,5° (c=1, Methanol).

**8.** Arzneimittel enthaltend eine oder mehrere Verbindungen nach Anspruch 1 zusammen mit den üblichen pharmazeutischen Hilfs- und/oder Trägerstoffen.

**9.** Verwendung von Verbindungen nach Anspruch 1 zur Herstellung von Arzneimitteln für die Behandlung von Atemwegserkrankungen und/oder Dermatosen.

**10.** Verwendung von Verbindungen nach Anspruch 1 zur Herstellung von Arzneimitteln für die Behandlung von Bluthochdruckerkrankungen verschiedener Genese und den damit zusammenhängenden Begleiterkrankungen.

## Claims

**1.** Compounds of formula I,

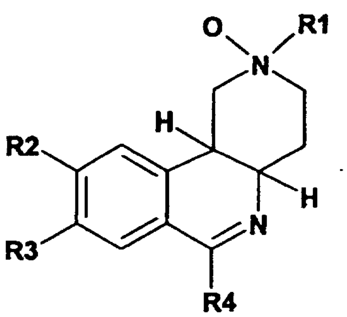

(I)

in which

R1 is 1-4C-alkyl,
R2 is hydroxyl, 1-4C-alkoxy, 3-7C-cycloalkoxy, 3-7C-cycloalkylmethoxy or 1-4C-alkoxy which is completely or predominantly substituted by fluorine,
R3 is hydroxyl, 1-4C-alkoxy, 3-7C-cycloalkoxy, 3-7C-cycloalkylmethoxy or 1-4C-alkoxy which is completely or predominantly substituted by fluorine,

or in which
R2 and R3 together are a 1-2C-alkylenedioxy group,

R4 is a phenyl radical which is substituted by R5, where
R5 is a tetrazol-5-yl radical which is substituted by a radical R6, where
R6 is hydrogen, 1-10C-alkyl, 3-7C-cycloalkyl, 3-7C-cycloalkylmethyl or Ar-1-4C-alkyl, where
Ar is a phenyl radical which is unsubstituted or substituted by R7 and/or R8, and

R7 and R8 independently of one another are 1-4C-alkyl or 1-4C-alkoxy,
and the salts of these compounds.

**2.** Compounds of formula I as claimed in claim 1, in which

R1 is 1-2C-alkyl,
R2 is 1-4C-alkoxy, 3-7C-cycloalkoxy, 3-7C-cycloalkylmethoxy or 1-4C-alkoxy which is completely or predominantly substituted by fluorine,
R3 is 1-4C-alkoxy, 3-7C-cycloalkoxy, 3-7C-cycloalkylmethoxy or 1-4C-alkoxy which is completely or predominantly substituted by fluorine, or in which

R2 and R3 together are a 1-2C-alkylenedioxy group,

R4 is a phenyl radical which is substituted by R5, where
R5 is a tetrazol-5-yl radical which is substituted by a radical R6, where
R6 is hydrogen, 1-7C-alkyl, 3-7C-cycloalkyl, 3-7C-cycloalkylmethyl or Ar-1-4C-alkyl, where
Ar is a phenyl radical which is unsubstituted or substituted by R7 and/or R8, and

R7 and R8 independently of one another are 1-4C-alkyl or 1-4C-alkoxy,
and the salts of these compounds.

**3.** Compounds of formula I as claimed in claim 1, in which

R1 is methyl,
R2 is 1-4C-alkoxy, 3-7C-cycloalkoxy, 3-7C-cycloalkylmethoxy or 1-2C-alkoxy which is completely or predominantly substituted by fluorine,
R3 is 1-4C-alkoxy, 3-7C-cycloalkoxy, 3-7C-cycloalkylmethoxy or 1-2C-alkoxy which is completely or predominantly substituted by fluorine,
R4 is a phenyl radical which is substituted by R5, where
R5 is a tetrazol-5-yl radical which is substituted by a radical R6, where
R6 is hydrogen, 1-7C-alkyl, 5-7C-cycloalkyl, 3-7C-cycloalkylmethyl or Ar-1-2C-alkyl, where
Ar is a phenyl radical which is unsubstituted or substituted by R7, and
R7 is 1-2C-alkyl or 1-2C-alkoxy,

and the salts of these compounds.

**4.** Compounds of formula I as claimed in claim 1, in which

R1 is methyl,
R2 is 1-4C-alkoxy,
R3 is 1-4C-alkoxy,
R4 is a phenyl radical which is substituted by R5, where
R5 is a tetrazol-5-yl radical substituted by a radical R6, where
R6 is hydrogen, 1-7C-alkyl, cyclohexylmethyl or 4-methoxybenzyl,

and the salts of these compounds.

**5.** Compounds of formula I as claimed in claim 1, in which

R1 is methyl,
R2 is ethoxy,
R3 is methoxy or ethoxy,
R4 is a phenyl radical which is substituted by R5, where

R5 is a tetrazol-5-yl radical substituted by a radical R6, where
R6 is 1-4C-alkyl,

and the salts of these compounds.

**6.** Compounds of formula I as claimed in claim 1, in which the hydrogen atoms in positions 4a and 10b are in the cis position relative to one another, or the salts of these compounds.

**7.** Compounds of formula I as claimed in claim 1, which in the positions 4a and 10b has the same absolute configuration as the compound (-)-cis-4-amino-3-(3-ethoxy-4-methoxyphenyl)-1-methylpiperidine dihydrochloride having the optical rotation $[\alpha]_D^{20}$ = -65.5° (c=1, methanol), which can be employed as a starting material.

**8.** A medicament comprising one or more compounds as claimed in claim 1 together with customary pharmaceutical excipients and/or vehicles.

**9.** The use of compounds as claimed in claim 1 for the production of medicaments for the treatment of airway disorders and/or dermatoses.

**10.** The use of compounds as claimed in claim 1 for the production of medicaments for the treatment of high blood pressure disorders of various origins and the accompanying disorders associated therewith.

## Revendications

**1.** Composés de la formule 1 :

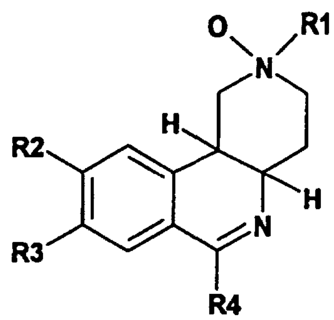

(I)

dans laquelle :

R1 représente un alkyle en $C_{1-4}$ ;
R2 représente un hydroxy, un alcoxy en $C_{1-4}$, un cycloalcoxy en $C_{3-7}$, (un cycloalkyle en $C_{3-7}$)méthoxy ou un alcoxy en $C_{1-4}$ complètement ou de manière prédominante substitué par du fluor ;
R3 représente un hydroxy, un alcoxy en $C_{1-4}$, un cycloalcoxy en $C_{3-7}$, (cycloalkyle en $C_{3-7}$)méthoxy ou un alcoxy en $C_{1-4}$ complètement ou de manière prédominante substitué par du fluor ;

ou dans laquelle :
R2 et R3 représentent ensemble un radical alkylènedioxy en $C_{1-2}$,

R4 représente un radical phényle substitué par R5, où
R5 représente un reste tétrazol-5-yle substitué par un reste R6, où
R6 représente un hydrogène, un alkyle en $C_{1-10}$, un cycloalkyle en $C_{3-7}$, un (cycloalkyle en $C_{3-7}$)méthyle ou un Ar(alkyle en $C_{1-4}$), où
Ar représente un radical phényle non substitué ou substitué par R7 et/ou R8, et

R7 et R8 représentent indépendamment l'un de l'autre, un alkyle en $C_{1-4}$ ou un alcoxy en $C_{1-4}$, ainsi que les sels de ces composés.

**2.** Composés de la formule I selon la revendication 1, dans lesquels :

R1 représente un alkyle en $C_{1-2}$ ;
R2 représente un alcoxy en $C_{1-4}$, un cycloalcoxy en $C_{3-7}$, un (cycloalkyle en $C_{3-7}$)méthoxy ou un alcoxy en $C_{1-4}$ complètement ou de manière prédominante substitué par du fluor;
R3 représente un alcoxy en $C_{1-4}$, un cycloalcoxy en $C_{3-7}$, un (cycloalkyle en $C_{3-7}$)méthoxy ou un alcoxy en $C_{1-4}$ complètement ou de manière prédominante substitué par du fluor;

ou dans laquelle :
R2 et R3 représentent ensemble un radical alkylènedioxy en $C_{1-2}$,

R4 représente un radical phényle substitué par R5, où
R5 représente un reste tétrazol-5-yle substitué par un reste R6, où
R6 représente un hydrogène, un alkyle en $C_{1-7}$, un cycloalkyle en $C_{3-7}$, un (cycloalkyle en $C_{3-7}$)méthyle ou un Ar(alkyle en 1-4C), où
Ar représente un radical phényle non substitué ou substitué par R7 et/ou R8, et

R7 et R8 représentent indépendamment l'un de l'autre un alkyle en $C_{1-4}$ ou un alcoxy en $C_{1-4}$,
ainsi que les sels de ces composés.

**3.** Composés de la formule I selon la revendication 1, dans lesquels :

R1 représente un méthyle ;
R2 représente un alcoxy en $C_{1-4}$, un cycloalcoxy en $C_{3-7}$, un (cycloalkyle en $C_{3-7}$)méthoxy ou un alcoxy en $C_{1-2}$ complètement ou de manière prédominante substitué par du fluor,
R3 représente un alcoxy en $C_{1-4}$, un cycloalcoxy en $C_{3-7}$, un (cycloalkyle en $C_{3-7}$)méthoxy ou un alcoxy en $C_{1-2}$ complètement ou de manière prédominante substitué par du fluor;
R4 représente un radical phényle substitué par R5, où
R5 représente un reste tétrazol-5-yle substitué par un reste R6, où
R6 représente un hydrogène, un alkyle en $C_{1-7}$, un cycloalkyle en $C_{5-7}$, un (cycloalkyle en $C_{3-7}$)méthyle ou un Ar(alkyle en $C_{1-2}$), où
Ar représente un radical phényle non substitué ou substitué par R7, et
R7 représente un alkyle en $C_{1-2}$ ou un alcoxy en $C_{1-2}$,

ainsi que les sels de ces composés.

**4.** Composés de la formule I selon la revendication 1, dans lesquels :

R1 représente un méthyle ;
R2 représente un alcoxy en $C_{1-4}$ ;
R3 représente un alcoxy en $C_{1-4}$ ;
R4 représente un radical phényle substitué par R5, où
R5 représente un reste tétrazol-5-yle substitué par un reste R6, où
R6 représente un hydrogène, un alkyle en $C_{1-7}$, un cyclohexylméthyle ou un 4-méthoxybenzyle,

ainsi que les sels de ces composés.

**5.** Composés de la formule I selon la revendication 1, dans lesquels :

R1 représente un méthyle ;
R2 représente un éthoxy ;
R3 représente un méthoxy ou un éthoxy ;
R4 représente un radical phényle substitué par R5, où
R5 représente un reste tétrazol-5-yle substitué par un reste R6, où
R6 représente alkyle en $C_{1-4}$,

ainsi que les sels de ces composés.

**6.** Composés de la formule I selon la revendication 1, dans lesquels les atomes d'hydrogène en positions 4a et 10b sont en position cis l'un par rapport à l'autre, ainsi que les sels de ces composés.

**7.** Composés de la formule I selon la revendication 1, qui ont la même configuration absolue en les positions 4a et 10b, comme le composé pouvant être mis en oeuvre comme produit de départ, le dichlorhydrate de (-)-cis-4-amino-3-(3-éthoxy-4-méthoxyphényl)-1-méthylpipéridine avec la valeur de rotation optique $[\alpha]^{20}_D$ = -65,5° (c = 1, méthanol).

**8.** Agent pharmaceutique contenant un ou plusieurs composés selon la revendication 1, avec les auxiliaires et supports pharmaceutiques usuels.

**9.** Utilisation des composés selon la revendication 1, pour la préparation d'agents pharmaceutiques pour le traitement de maladies des voies respiratoires et/ou de dermatoses.

**10.** Utilisation des composés selon la revendication 1, pour la préparation d'agents pharmaceutiques pour le traitement de maladies de l'hypertension de différentes genèses et des maladies dépendantes.